(19)

**Europäisches Patentamt**
**European Patent Office**
**Office européen des brevets**

(11) **EP 4 239 538 A2**

(12) **EUROPEAN PATENT APPLICATION**

(43) Date of publication:
**06.09.2023  Bulletin 2023/36**

(21) Application number: **23181450.0**

(22) Date of filing: **29.07.2019**

(51) International Patent Classification (IPC):
***G06N 20/00*** *(2019.01)*

(52) Cooperative Patent Classification (CPC):
**G06N 20/20; A61C 7/00; A61C 7/002; G06N 3/045;
G06N 3/088;** G06N 5/01; G06N 7/01; G06N 20/10

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **31.07.2018   US 201862712383 P**

(62) Document number(s) of the earlier application(s) in
accordance with Art. 76 EPC:
**19844870.6 / 3 829 481**

(71) Applicant: **3M Innovative Properties Company**
**St. Paul, MN 55133-3427 (US)**

(72) Inventors:
 • **BEN-GAL NGUYEN, Nitsan**
   **Saint Paul, MN 55133-3427 (US)**
 • **ZIMMER, Benjamin D.**
   **Saint Paul, MN 55133-3427 (US)**
 • **SOMASUNDARAM, Guruprasad**
   **Saint Paul, MN 55133-3427 (US)**
 • **CUNLIFFE, Alexandra R.**
   **Saint Paul, MN 55133-3427 (US)**
 • **STANKIEWICZ, Brian**
   **Saint Paul, MN 55133-3427 (US)**
 • **COLLINS, Elisa J.**
   **Saint Paul, MN 55133-3427 (US)**
 • **MAIDEN MUELLER, David T.**
   **Saint Paul, MN 55133-3427 (US)**

 • **DEMLOW, Steven C.**
   **Saint Paul, MN 55133-3427 (US)**
 • **OLSON, Cody J.**
   **Saint Paul, MN 55133-3427 (US)**
 • **AFRIDI, Muhammad J.**
   **Saint Paul, MN 55133-3427 (US)**
 • **AMATO, Nancy**
   **Champaign, IL 61822 (US)**
 • **THOMAS, Shawna L.**
   **College Station, TX 77845 (US)**
 • **GUTIERREZ, Alexander**
   **Minneapolis, MN 55408 (US)**
 • **SANGARI, Arash**
   **Frisco, TX 75033 (US)**
 • **GANDRUD, Jonathan D.**
   **Saint Paul, MN 55133-3427 (US)**

(74) Representative: **Vossius & Partner**
**Patentanwälte Rechtsanwälte mbB**
**Siebertstraße 3**
**81675 München (DE)**

Remarks:
•This application was filed on 26-06-2023 as a
divisional application to the application mentioned
under INID code 62.
•Claims filed after the date of filing of the application
/ after the date of receipt of the divisional application
(Rule 68(4) EPC).

(54) **METHOD FOR AUTOMATED GENERATION OF ORTHODONTIC TREATMENT FINAL SETUPS**

(57)    The present invention relates to a computer-implemented method of generating a final setup for orthodontic treatment using supervised machine learning for direct mapping, comprising receiving, by one or more computer processors, a training set of patient case data which includes malocclusion states and ground truth images representing final setup states for the teeth of one or more patients, wherein the final setup states represent orthodontic post-treatment positions of a person's teeth and the malocclusion states are pre-treatment positions of the teeth and are represented by digital 3D models of teeth in an initial state, generating, by the one or more computer processors using a deep neural network, a generated output image, computing, by the one or more computer processors, a distance between a generated output image and the ground truth image representing the final state using a loss function, and minimizing, by the one or more computer processors, the distance to train, at least in part, the deep neural network.

EP 4 239 538 A2

## Description

### BACKGROUND

The goal of the orthodontic treatment planning process is to determine where the post-treatment positions of a person's teeth (setup state) should be, given the pre-treatment positions of the teeth in a malocclusion state. This process is typically performed manually using interactive software and is a very time-consuming process. A need thus exists for a final setup algorithm to serve as an efficiency enhancer, greatly reducing the time needed to process a case, along with a way to automatically rate and evaluate treatment options.

### SUMMARY

A computer-implemented method for generating one or more final setups for orthodontic treatment of an embodiment of the invention includes receiving a digital 3D model of teeth in an initial state.

The method computes a scoring function based upon metrics related to the initial state to generate a corresponding score, perturbs a state of the teeth through movement of a tooth or set of teeth within the digital 3D model of teeth to generate a perturbed state of the teeth, and updates the state of the teeth based upon the score and the perturbed state of the teeth to generate one or more final setups representing a final state of the teeth after the orthodontic treatment.

Another computer-implemented method of an embodiment of the invention generates metrics for use in the scoring function to generate a final setup and can rate final setups.

### BRIEF DESCRIPTION OF THE DRAWINGS

The accompanying drawings are incorporated in and constitute a part of this specification and, together with the description, explain the advantages and principles of the invention. In the drawings,

FIG. 1 is a diagram of a system for automated generation of orthodontic treatment final setups;
FIG. 2 is a flow chart of a method for automated generation of orthodontic treatment final setups;
FIG. 3 is a diagram of a tooth coordinate system for use in computing metrics; and
FIG. 4 is a diagram of an arch form coordinate system for use in computing metrics.

### DETAILED DESCRIPTION

#### Overview

The purpose of the final setups algorithm is to achieve final positions of teeth that are aesthetically and functionally correct according to best practices and orthodontists' opinions. Setup technicians and/or orthodontists can use orthodontic rules, for example, when deciding the final positions of teeth. These rules can be captured using metrics such as overbite, overjet, leveling, alignment, and others. By defining acceptable levels of these various metrics the system can model what is a good setup. Acceptable levels can be set through a combination of domain knowledge and thresholds inferred from data of historical final setups.

Teeth can be modeled as objects in three-dimensional (3D) space that have movement restrictions for certain types of motion. By converting heuristics into objective metrics, the problem of finding final setups can be formulated as an optimization problem where the goal is to get as many of the orthodontic metrics to acceptable levels as possible while significantly satisfying tooth motion limits. In general, the goal is to achieve the best possible end state using these metrics without exceeding limits on arch form changes, extractions, crown movement, root movement, and IPR (interproximal reduction, also known as teeth shaving).

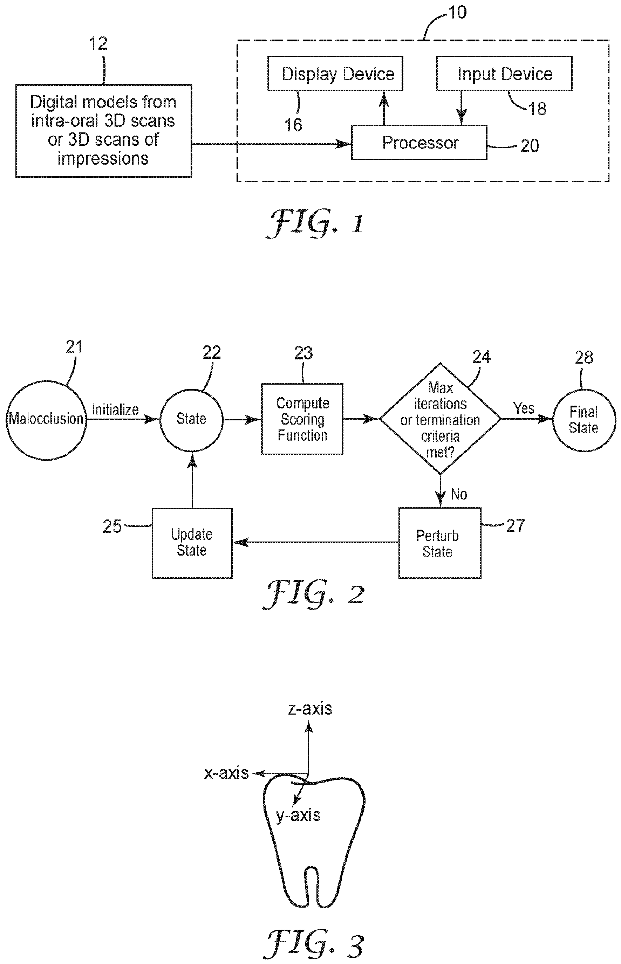

FIG. 1 is a diagram of a system 10 for receiving and processing digital 3D models based upon intra-oral 3D scans. System 10 includes a processor 20 receiving digital 3D models of teeth (12) from intra-oral 3D scans or scans of impressions of teeth. System 10 can also include an electronic display device 16, such as a liquid crystal display (LCD) device, and an input device 18 for receiving user commands or other information. Systems to generate digital 3D images or models based upon image sets from multiple views are disclosed in U.S. Patent Nos. 7,956,862 and 7,605,817, both of which are incorporated herein by reference as if fully set forth. These systems can use an intra-oral scanner to obtain digital images from multiple views of teeth or other intra-oral structures, and those digital images are processed to generate a digital 3D model representing the scanned teeth. System 10 can be implemented with, for example, a desktop, notebook, or tablet computer. System 10 can receive the 3D scans locally or remotely via a network.

**I. Final Setup Algorithm**

Inputs to final setup algorithm:

- Tooth positions for every tooth in maloccluded (initial) state represented using position and orientation in the arch form coordinate system. Tooth positions can be obtained, for example, by segmenting a digital 3D model of teeth into individual point structures for each tooth. A collection of these coordinates, along with the mesial and distal interproximal reduction (IPR) amounts for all the teeth, is considered a state.
- Information about tooth landmarks and coordinate systems.
- Information about extractions - teeth to be removed.
- Information about holding/fixing teeth during treatment - these could be implants, molars or other teeth.
- Information about desired or allowed IPR.
- Information about limits on tooth movements

Additional optional inputs to final setup algorithm:

- Information about areas of focus

- Information about orthodontic qualities that should be maintained or improved

- Information about desired treatment duration

- Information about desired tooth or arch form changes

Output of the final setup algorithm:

- Positions, orientations, and IPR quantities for the teeth in the arch form coordinate system that represent one or more Final Setup States. If IPR was deemed necessary it would have been applied to the desired teeth.

FIG. 2 is a flow chart of a method for automated generation of orthodontic treatment final setups. The method of FIG. 2, as further described below, can be implemented in software or firmware modules, for example, for execution by processor 20 or other computing device, and can alternatively be implemented in hardware modules or a combination of software and hardware.

The main components of the algorithm are the scoring function 23, the perturbation and update processes 25 and 27, and the optional initializer 21. The initialization steps alone could be used to create a final setup, however it may be suboptimal compared to one created with the full optimization workflow.

The adjusted or updated states can serve as a rough sketch or an approximate that an expert such as an orthodontist can provide feedback or improve upon. Several adjusted states can be provided as options to choose from. Alternatively, a partially optimized setup instead of a fully optimized setup can serve the same purpose.

Scoring function (23):

At the core of the algorithm is a scoring function or an objective function that the algorithm attempts to minimize.

$$Score(X) = \sum_{i=1}^{n\_metrics} w_i P_i(x_i)$$

The equation above indicates a general form of the scoring function. Here X represents the vector of metrics computed for a state. $P_i$ is a penalty function that computes the error or penalty given a value of a metric and acceptable levels for that metric. For instance this penalty could be the absolute difference between the median of acceptable values of this metric and the current instance value, or the squared difference, and so on. Thus w; is the weight associated with the penalty for that metric, which is a scalar quantity. The weights w allow the scoring function to weight different metrics more or less, making it possible to generate multiple scoring functions that emphasize different orthodontic metrics and allow the system or user to select the scoring function from a pool of scoring functions, or have the scoring functions change as the teeth are being perturbed. For example, one scoring function might focus on overall correctness, while another would focus on tooth alignment. Weights can be predetermined based on clinical or geometric rules or learned from existing malocclusions and/or final setup data. Alternatively, weights can be computed based on doctor or technician input specifying areas of focus (e.g., incisor alignment), treatment objectives (e.g., good bite), or case-specific preferences (e.g., maintaining space between teeth).

In another embodiment, the function *P*, instead of representing a penalty, can be configured to represent how close a value of the metric is to an ideal or acceptable value for the metric.

A general form of the scoring function is f(P(x)) where f can be any function including but not limited to linear, non-linear, or a probabilistic framework such as machine learning classifiers. As an example, it can be simple weighted combination shown in the equation.

Another option is to dynamically select the weights over various iterations by identifying greatest opportunities for improvement. This option can use boosting where instead of assigning weights on samples or records, the weights are associated with individual metrics. This option can also use the boosting algorithm where the weights are dynamically adjusted to put more emphasis on metrics/scores that have maximum room for improvement. It should be noted that the metrics can change as the states change. Additionally, by using a scoring function that penalizes collisions, inter-penetrations, or gaps between teeth as teeth are moved, the algorithm can resolve these issues. This allows the use of the same optimizer framework to resolve collisions and gaps as well as improve orthodontic correctness of a state by switching out the scoring functions appropriately.

An optional regularizing function can also be applied to ensure that, by improving one scoring function, other potentially competing scoring functions do not worsen by a given amount. For example, the algorithm could be run with an edge alignment scoring function plus a competing collision scoring function that would prevent the optimizer from improving the edge alignment score by moving teeth into deep collision with one another.

Metrics Used in The Scoring Function:

A detailed description of orthodontic metrics is provided in co-owned US patent application filed on even date here entitled, "Dashboard for Visualizing Orthodontic Metrics During Setup Design" (U.S. Provisional Application No. 62/712380, filed July 31, 2018), which is incorporated herein by reference as if fully set forth.

Perturb State (27):

Perturb state is a set of functions that allow physical transformations in 3D which comprise translations and rotations of an individual tooth or a set of teeth and IPR. The sets can be randomly chosen, they can be semantically chosen groups such as the social six (incisors, canines) or posterior set, or they can be selected in a directed way such as selecting teeth that have the best opportunity of improving a scoring function. Many perturbations can be applied and evaluated, with the optimal one (as chosen by optimizing a score or via an iterative optimization algorithm such as gradient descent described in the next section) being selected. Perturbations are subject to motion constraints to prevent unrealistic tooth movement. To enforce these constraints, positions of perturbed teeth are modified to remain within movement limits.

Gradient Computation:

One method of perturbing is through gradient descent, where a numerical gradient is computed and used to minimize the scoring function. Adjusting a state by rotation or translation or a combination thereof in one direction and in the opposite direction yields new states. By computing the difference between the scoring function evaluations at these states, a numerical gradient for the orthodontia metrics along the direction can be computed. This process can be repeated for multiple directions to yield multiple difference values, which can be aggregated to approximate the gradient direction of the function at the current state. Multiple directions with large differences can be stored and subsequently explored. Gradient computation can be applied for one tooth at a time or groups of teeth. Alternatively, methods such as Levenberg-Marquardt can optimize a vector-valued objective function to generate new states.

Given the gradient direction and the state, perturbed states are created by moving different distances along the gradient direction. Historic gradient directions from past iterations of the optimization algorithm may be stored and used to inform future gradient directions or gradient directions for neighboring teeth. In addition to historic gradient directions, perturbed states can also be stored especially if there are states with very close scoring function values. A state from multiple stored states can be chosen using a sampling process. Alternatively, multiple perturbed states can be chosen from using a probabilistic process.

Update State (25):

By repeatedly updating the state through selecting scoring functions, perturbing, and selecting states that decrease the value of the scoring function, an improved final state can be generated after a chosen maximum number of iterations, if the scoring function evaluations are close to optimal (metrics are within acceptable levels), or if the scoring function is no longer changing by more than a small delta amount (indicating that it has reached a local or global minimum). Termination criteria can be defined on scores using a separate set of weights for the metrics which represent the relative importance of metrics for orthodontic correctness. Optimization can also be terminated if the scoring function value does not change significantly determined by a threshold over a few predetermined number of iterations.

Initialization (interproximal reduction) (21 and 22):

IPR initialization determines whether IPR should be applied to each tooth. IPR can either be prescribed clinically (e.g., based on a doctor prescription or technician input) or determined automatically. Automated IPR prediction is performed by computing the amount of space available in the arch under a set of transformations. If the space is less than a predetermined threshold amount, IPR is applied to create space. This IPR amount can be calculated by optionally varying the arch form shape (typically expanding) and computing the amount of IPR required for each arch form shape scenario.

Initialization (Tooth Geometry) (21 and 22):

Some of the orthodontic metrics can be optimized using fast direct transformations ahead of the optimizer. Tooth geometry initialization is the process of applying some transformations to the malocclusion that are either input manually (e.g., by a doctor or technician), predetermined based on clinical or geometric rules, or predicted by machine learning algorithms trained from historically generated manual setups. An effective initialization takes the positions of the teeth as close to ideal as possible, thereby reducing the number of optimization iterations required to reach a final state that is close to optimal. Teeth can be randomly transformed from their malocclusion state but this method doesn't offer any guarantees of optimality. Details of various initialization techniques are described as follows:

Unrotate:

This initialization technique eliminates any rotation with respect to the arch form coordinate system for each individual tooth.

Leveling:

The leveling technique repeatedly applies transformations to teeth so that the leveling score is improved. The leveling score is a measure of the relative heights of neighboring teeth.

Symmetric Translations/Rotations:

Rotations and translations can be adjusted to create left/right symmetry. Symmetric translation and rotation amounts may be set to pre-determined ideal values that have been determined heuristically or learned from data. Alternatively, current translation/rotation amounts for symmetric tooth pairs in a state may be used to derive new symmetric values for translation/rotation. For instance, the system can compute the mean, minimum, or maximum rotation amount for symmetric tooth pairs and set the rotation for both teeth to this value. One example of a symmetric translations initializer is one that computes distance along the arch form from the midline to each member of a symmetric tooth pair and sets the tooth positions for this pair of teeth to the mean, minimum, or maximum of the two computed distances.

Parallel Arches:

This initialization technique attempts to make the arch forms of the upper and lower arch parallel by iteratively moving teeth along the axis perpendicular to each arch form. This process continues until either the maximum number of iterations is reached or until the standard deviation of inter-arch distances between teeth on one arch and their nearest neighbors on the opposing arch drops below a threshold.

Ideal Rotation:

Ideal rotation amounts around tooth x, y, and z axes can be determined heuristically or learned using historic data. This initialization technique applies the ideal rotation amount for each individual tooth.

Ideal Arch Form Shape:

Ideal arch form shapes may be modeled after geometric functions (e.g., parabola, hyperbola, geometric spline, or catenary curve) or learned from historic data. During initialization, tooth positions are adjusted to conform to these ideal shapes. Scaling may be used to account for differences in overall arch form length between individual cases.

Expansion:

In cases where insufficient space exists in an arch to create a setup with teeth aligned along the arch form, an expansion adjuster can be applied to create space. Expansion can be achieved through a combination of translation normal to the arch form and flare (rotation in the labial/lingual direction with center of rotation at or near the tooth root).

Anti-Mesialization and Distalization:

Adjustments can also be made after optimization. One example is for mitigating unwanted movement of the entire set of teeth along the front-to-back axis of the mouth during optimization. This technique averages the position of all the molars along the front-to-back axis, and after optimization, moves the entire set of teeth into such a position as to produce the same average position for the molars.

Edge/Groove Alignment:

This initializer aligns the teeth along the arch form and rotates them so that the front-back axis of each tooth is pointed along the direction normal to the arch form. Teeth may be aligned based on the position of incisal edges, cusp tips, molar/pre-molar grooves, or a combination of these locations.

Class Relationship:

This initializer adjusts whole arch translation and molar & pre-molar rotation to correct class relationship.

Crossbite/Overjet:

This initializer translates teeth in a direction normal to the arch form to correct crossbite and improve overjet.

Packing or gap closing:

In cases where there are gaps in the arch form between teeth, an algorithm to pack teeth tightly may save time during optimization.

Direct Mapping and Machine Learning Methods:

Supervised approaches for direct mapping: Given a training set of cases which include the malocclusion and final setup states, machine learning models can be developed in order to model the mapping between malocclusion and final states directly in the state space, transformation space, metric space, or a combination thereof. For example, deep neural network based approaches such as auto-encoders or more specifically convolutional auto-encoders could be utilized for this purpose. In case of convolutional auto-encoders (CAE), the input space (representing malocclusion state) can be simply reshaped to an image representation and then the hidden layers of the CAE could act as a transformation function to transform this image into an output image that is the representative of the final state. Each hidden layer applies 2D convolutional filters learned during the training stage. This is usually followed by applying non-linear function (such as Rectified Linear Units) to the output. The output of each hidden layer is then passed to the next hidden layer. The final output is an image of the same size as that of an input and represents the final state. In this case, a loss function such a Euclidean loss could be utilized during the training phase. Such a loss tries to minimize the distance between the generated output image and the ground truth image representing the final state.

Principal Component Analysis (PCA) based reconstruction could also be utilized in the same manner for direct mapping. The approach will take the input representing malocclusion state and try to reconstruct the corresponding final state. Using Euclidean loss will still be valid. However, PCA based approaches are based on linear transformations and therefore, have limited ability to learn complex transformations. On the other hand, deep learning neural network based approaches could learn these complex transformations within multiple hidden layers.

Clustering based unsupervised approaches: The goal of clustering-based methods is to assign a given state (in whole or in part) to clusters of other "similar" mouth states and then use those clusters to determine how to change the position of teeth. Examples of features that might be used for clustering include the rotations of each tooth in the arch form coordinate system (in some suitable representation such as quaternions), the absolute positions of the teeth in space, or any of the metrics described previously. The clustering algorithm can range from standard algorithms such as k-means to new ideas such as using a deep neural network autoencoder to change the given features into a given "feature space" and then doing the clustering in that feature space. Clustering can be used to learn either the absolute position of teeth, or the relative motion that teeth should undergo:

1. Absolute clustering motion: given a maloccluded state $A$ and an element $B$ of the cluster $C_1$ to which that state $A$ (in whole or in part) is assigned, the system could simply replace some aspect or property of the maloccluded mouth $A$ with the aspect or property of the assigned state (e.g. the corresponding aspect of the associated final state to $B$, or an idealized state) in the correct final state cluster $C_1^*$.
2. Relative clustering motion: given a maloccluded state MS1 and another maloccluded state MS2 which is in the same cluster (with respect to certain features) and for a final setup state (FS2) the system could apply the same motion to the maloccluded mouth that was applied to get from MS2 -> FS2 to MS1.

Combination approaches:

A set of initialization methods may be applied in series. For example, empirically good results have been produced by performing a clustering-based method followed by leveling, parallel arch form adjustment, and symmetric translations along the arch form.

Once the maximum iterations or termination criteria are met (24), the final state (28) is generated for the final setup. The final setup can be used, for example, to generate intermediate setups to make clear tray aligners to incrementally move the teeth from the initial or maloccluded state to the final state represented by the final setup.

## II. Metrics and Scores for Final Setups

Described herein are methods to automatically rate final setups for orthodontic treatment. These methods can be used in an interface to aid doctors and technicians during final setup creation and/or evaluation, or they can be used in an automated process to create or select final setups. First, a set of metrics are computed from one or several candidate final setups. These metrics can then be used directly, or they can be converted into scores that reflect the deviation of the value of each metric from population averages. Alternatively, machine learning can be used to derive a combination of the metrics that differentiate final setups from malocclusions and other non-ideal arrangements of teeth. Metrics are listed and defined in the table below.

Table 1. List of metrics and brief explanations

| | |
|---|---|
| Alignment | Orientation of a tooth relative to the local arch form orientation. |
| Edge alignment | Alignment of molar and bicuspid cusp tips with canine tips and incisor edges. |
| Buccolingual inclination | Molar buccolingual tilt on opposite sides of an arch. |
| Class relationship | Relative position of upper and lower arch molars and canines to each other. |
| Leveling | Relative tooth heights of neighboring teeth. |
| Mesial/distal tilt | Tooth tilt in the mesial/distal direction. |
| Torque | Rotation of teeth in the buccal/lingual direction. |
| Relative midline | Alignment between upper and lower arch central incisors. |
| Absolute midline | Position of the central incisors with respect to the midline of a patient's face. |
| Occlusal contacts | Distance from cusps to teeth in opposing arch. |

(continued)

| Interarch contacts | Upper and lower arch contacts/collisions. Occlusal contacts refer to pre-molars and molars contacting between upper and lower arch, and incisor and canine contacts refer to contact/spacing between anterior teeth. |
|---|---|
| Overbite | Vertical overlap between upper arch central incisors and lower arch central incisors relative to incisal ridges. |
| Overjet | Protrusion of upper arch central incisors with respect to lower arch central incisors, i.e. horizontal overlap of upper central incisors over lower central incisors. |
| Crossbite | The buccal cusp tips of lower arch molars should be positioned directly below the central grooves of the upper arch molars. |
| Canine overbite | Overlap between upper and lower arch canines. |
| Spacing | Distance between teeth along an arch form. |
| Collision | Overlap (interpenetration) between colliding tooth meshes. |
| Contact | A measurement of collisions and gaps between neighboring teeth. |
| Bite symmetry | Symmetry of tooth overlap with the occlusal plane. |
| Arch form shape | Measurement of how well the overall shape of the teeth is described by a model family of arch forms |
| Arch form symmetry | Symmetry of arch form shape on the left and right sides of the midline |
| Arch form expansion | Individual tooth wise arch form expansion can be measured as the displacement in the d direction from the malocclusion to a specific state |
| Arch form parallelism | Teeth on the upper and lower arch are positioned such that the distance between each tooth and its neighbors on the opposite arch is the same for every tooth in the arch. |
| Arch form ratio | Ratio of upper and lower arch length to corresponding lengths of the malocclusion |
| Golden proportions and Bolton ratio | Measure of tooth widths or sum of tooth widths to represent orthodontic norms of aesthetic smiles |
| Curve of spee | A measure of curvature of mandibular arch form that represents optimal vertical arrangement of teeth. Also captured implicitly using leveling metric. |

**A: Computation of metrics**

Pre-processing steps:

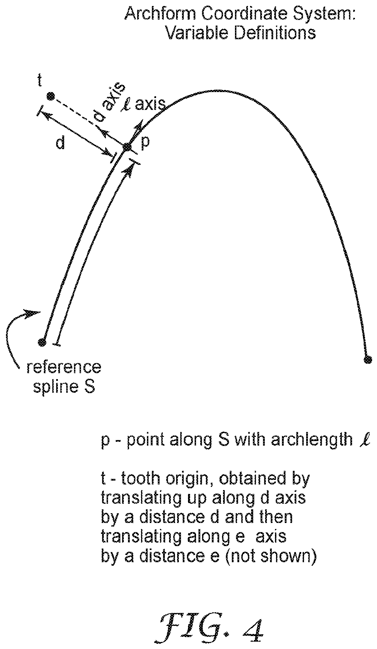

Several metrics are computed based on tooth coordinate systems and/or landmarks that have been defined on the teeth. Tooth coordinate systems are defined as shown in FIG. 3. Other metrics are computed based on an arch form coordinate system. The arch form coordinate system is shown in FIG. 4. Based on these coordinate systems, metrics can be computed as follows.

Alignment - Each tooth has a coordinate system that was defined either automatically or by a technician. For each tooth, compute the absolute angle between the X axis of the tooth's coordinate system and a tangent vector. This tangent vector is the tangent of a curve that approximates the form of the arch and is located at the location of the tooth origin in global coordinates. The curve is computed using features of an arrangement of teeth; for instance, the curve could be a spline that uses the tooth centers as control points. The Alignment Metric reports the following (though other statistics are possible):

    List of all Angles
    Mean of Angles
    Median of Angles
    Standard Deviation of Angles

Edge alignment - For each pair of neighboring teeth, an edge alignment error function is calculated on four points that approximate the left and right corners of the incisal edge of each tooth. Many edge alignment error functions are possible, including:

- absolute difference of intercepts of lines fit to the mean-shifted pairs of left and right corner points

- 1 minus the absolute value of the dot product of two unit vectors: one constructed from both left points and one constructed from one right point and one left point

Buccolingual inclination - This score is described in the ABO Grading System for Dental Casts and Panoramic Radiographs. In exemplary software, it is implemented as measuring the distance from certain cusps to a plane which is defined by the z-axis and a line that passes through analogous cusps on opposite sides of the arch. For lower teeth, the plane is defined by the buccal cusps and the distance is measured from lingual cusps. For upper teeth, the plane is defined by the lingual cusps and the distance is measured from the buccal cusps. In the lower arch, this distance is calculated on the second bicuspids, first molars, and second molars. In the upper arch, this distance is calculated on the first bicuspids, second bicuspids, first molars, and second molars. It is only calculated on the mesial cusps of second molars.

Molar class relationship - Molar class relationship describes the positional relationship between upper arch cusp tips and lower arch grooves. Class relationship is defined separately for each molar (first, second, and third molars) and each side (left and right side). First, mesial buccal cusp tip locations are identified for upper arch molars and mesial, central, and distal buccal cusp tips are identified for lower arch molars. Next, lower arch grooves are approximated as the mean value of the mesial and central buccal cusp tips, or alternatively, as the mean value of the mesial and distal buccal cusp tips if the central cusp tips do not exist. Grooves may alternatively be better approximated using hill climbing. The molar class relationship metric is then computed as the distance between the upper arch cusp tip and the lower arch groove along the 1-axis:

<(upper_pt - lower_pt), l_axis>

Canine class relationship - Canine class relationship describes the positional relationship between upper arch canines with lower arch canines and bicuspids. Canine class relationship is defined separately for each side (left and right). First, upper and lower arch canine tips and lower arch bicuspid tips are identified either manually or using an automated approach such as hill climbing. Next, the central point between lower arch bicuspid and canine tips is computed by taking the mean of the two points. The metric is then computed as the distance between the mean point and the upper arch canine tip along the l-axis:

<(upper_pt - lower_pt), l_axis>

Leveling - Leveling describes the difference in tooth height between neighboring teeth. First, tooth origins are defined on the tip of each tooth (see Figure 1). Leveling is then computed as the difference between origin points of neighboring teeth along the z-axis:

<(o 1 - o2), z_axis>

Mesial/distal tilt - Mesial/distal tilt is computed as the rotation of the tooth around the 1-axis.

Relative midline - The relative midline metric examines the relative positions of the upper and lower central incisors. A representative point is then determined for each of the left and right corners of each tooth, along the incisal edge of the tooth. Then, the following midpoints are computed:

$$lower\_midline\_point = midpoint(left\_edge\_point\_LRC, right\_edge\_point\_LLC)$$

$$upper\_midline\_point = midpoint(left\_edge\_point\_URC, right\_edge\_point\_ULC)$$

where:

left_edge_point_LRC is the left incisal corner point of the lower right central incisor.
right_edge_point_LLC is the right incisal corner point of the lower left central incisor.
left_edge_point_URC is the left incisal corner point of the upper right central incisor.
right_edge_point_ULC is the right incisal corner point of the upper left central incisor.

Whether crowding has occurred on each arch is then evaluated, i.e. whether left_edge_point_LRC[0] < right_edge_point_LLC[0] and/or left_edge_point_URC[0] < right_edge_point_ULC[0] (positive x-direction going left, as determined by right hand rule). If crowding is occurring for the central incisors on a given arch, then the relative midline is chosen as the edge-point with maximal value in the y-direction. If no crowding is occurring, the relative midline can be computed as the absolute value of the difference in these midline values, where the difference is computed along a direction parallel to the X axis:

$$distancebetweenMidlines = abs(lower\_midline\_point[0] - upper\_midline\_point[0])$$

Absolute midline -The purpose of the absolute midline metric is to ensure that the midline, in addition to being consistent between the top teeth and the bottom teeth, is also aligned as much as possible with the middle and front of the mouth. The absolute midline metric is actually a family of metrics including:

1. The absolute value of the distance in the y direction between the central incisors. This is designed to measure how far the given teeth are from having the central incisors at the front of the mouth.
2. The difference between the midline points calculated as above and the vertex of a quadratic or biquadratic arch form fit to the teeth. See the "arch form shape" metric for more discussion on these arch forms.

Occlusal contacts - Measures the directed distance from the cusps of bicuspids and molars to the closest tooth surfaces in the opposing arch.

Overbite, Overjet, and Canine Overbite - Overbite is a measure of the degree to which teeth in the upper arch overshoot the teeth in the lower arch, along the Y axis, which runs from the back of mouth toward the front of the mouth. Overjet is a measure of the degree to which the teeth in the upper arch overshoot the teeth in the lower arch, along the Z axis, which points up out of the screen, when the teeth are viewed from above. Overbite is computed for the central incisors, canines and central bicuspids. Overjet is computed for the central incisors.

Overbite and Overjet can be computed as follows.

1) Compute overbite between upper and lower left central incisor by:

    a. compute difference of lower's peak vertex from upper's peak vertex for left central incisor (along Z axis)

2) Compute overjet between upper and lower left central incisor by:

    a. compute difference of upper and lower left central incisor (along Y axis)

3) Compute overbite between upper and lower right central incisor by:

    a. compute difference of lower's peak vertex from upper's peak vertex for right central incisor (along Z axis)

4) Compute overjet between upper and lower right central incisor by:

    a. compute difference of upper and lower right central incisor (along Y axis)

5) Compute overbite between upper and lower left canines and bicuspids by:

    a. compute average of:

        i. difference of upper's peak vertex from lower's peak vertex for left canines and
        ii. difference of upper's peak vertex from lower's peak vertex for left 1st bicuspids (along Z axis)

6) Compute overbite between upper and lower right canines and bicuspids by:

    a. compute average of:

        i. difference of upper's peak vertex from lower's peak vertex for right canines and
        ii. difference of upper's peak vertex from lower's peak vertex for right 1st bicuspids (along Z axis)

Spacing - Spacing describes the distance between neighboring teeth along the arch form. A spacing score is computed for each pair of neighboring teeth. First, adjacent tooth corners for neighboring teeth are identified (defined as the points that are the greatest distance from the tooth origin on the x-z plane). Next, the distance in the x-y plane between tooth corners of neighboring teeth is computed:

$$spacing = sqrt[(tooth1corner\_x – tooth2corner\_x)^{\wedge}2 + (tooth1corner\_y – tooth2corner\_y)^{\wedge}2]$$

Alternatively, spacing may be computed as the distance between tooth corners along the 1-axis:

$$spacing = <(tooth1\_corner – tooth2\_corner) , 1\_axis>$$

Collision - Methods to measure collisions include:

1) Penetration depth
2) Count of overlapping vertices between two meshes
3) Count of occluded vertices
4) Volume of occluded space (i.e., portions of the two meshes that overlap)
5) Surface are of the occluded portions of two meshes
6) Cross-sectional area of the portion of the plane of intersection that falls within the overlap of meshes

Contact - Computed for each pair of neighboring teeth meshes; if the meshes are in collision, the score is -1.0 multiplied by the penetration depth. If the teeth are not in collision, the score is positive and is the minimum distance between the meshes. A scalar value may be added to the score to bias it. For example, the system may add a small value to each score and attempt minimize the sum of absolute values of scores across the entire mouth, closing gaps and creating small collisions that are desirable for treatment plans with some overcorrection.

Bite symmetry - Bite symmetry describes the concept that tooth positions and relationships on the left and right sides of the arch should be similar to each other. To achieve this, the system computes a score that determines the dissimilarity of metrics for teeth on opposite sides of the arch. These metrics may include one of the metrics discussed above (e.g., torque, leveling) or a combination of these metrics (e.g., weighted sum of torque and leveling dissimilarity). Dissimilarity may be measured as the mean absolute difference between values, the mean absolute difference between each of these values and an ideal value, or the standard deviation of the values compared to an ideal value.

Arch form shape - Define the shape of the arch form to be the overall shape made by the teeth when projected onto the xy-plane. In general, this shape is formed by taking some function of the coordinates of the landmarks or mesh vertices in the global geometry. For example, one could use a point cloud where each point is the average of the x and y components of the mesh vertices for a single tooth. The arch form shape metric is a measure of how well the overall arch shape (in this case, the point cloud of up to 32 points) resembles (our model for) a good final setup shape. However, not all setup arch form shapes are the same. Therefore, computing the arch form metric is a two step process: first the system classifies a given arrangement of teeth as being closest to one of a family of arch form type. Then, given this arch "type" the system fits a highly regularized function (determined by the type) to the data and return some metric on the distance from the regularized function to the underlying data which was fit.

For example, a first step could be to train a classifier which will, given some mouth state, decide whether a mouth would be better represented by a quadratic or biquadratic arch form. Then, given this classification, the system will fit a polynomial of the given type (in this case, quadratic or biquadratic) to the data and return the residual of this polynomial fit as the metric.

Note that when using a symmetric family of functions to fit the data (e.g., quadratic or biquadratic) the system can also use a combination of the residual on either side of the arch, the difference between the vertex and the midpoint, as an alternative measure of the symmetry of the arch form. An alternative method of doing this is discussed below.

Arch form symmetry - The symmetry of the arch form shape may be computed by first calculating the absolute distance of each tooth origin from the midline, then measuring the difference in distances between identical teeth on opposite sides of the mouth. The distance may be measured as x and y distances separately, or as a single Euclidean distance.

Crossbite - A mouth with ideal crossbite would have the buccal cusp tips of the lower arch molars fit inside the central grooves of the upper arch molars. The steps to compute crossbite are shown below:

For each molar type (third molar, second molar, and first molar):

1. Identify the mesial and distal marginal ridge points of the tooth on the upper arch and compute the midpoint, m
2. Identify the central buccal cusp tip point, c, of the tooth on the lower arch
3. Compute the vector tangent, t, to the arch form at the upper arch tooth origin
4. Compute crossbite as the distance from m to c along the vector direction t:

$$crossbite = \langle (c - m), t \rangle$$

Inter-arch contact - Inter-arch contacts refers to the amount of penetration or spacing between neighboring teeth on the upper and lower arch. Refer to "Contact" for a description of how penetration and spacing are computed. If meshes in neighboring arches are in collision, the score is the penetration amount multiplied by -1.0. If the meshes are not in collision, the score is positive and is the minimum distance between meshes. Inter-arch contacts for each tooth individually can be used as metrics, or multiple metrics can be combined globally into a single metric per case or per tooth type. Methods for combining inter-arch contact metrics include a weighted linear or non-linear combination. Alternatively, an ideal set of contacts for a group of teeth can be learned from historic data and modeled by a parametrization such as a normal distribution or kernel density estimation. The score can be computed as the similarity of the current distribution to the ideal learned distribution using a function such as log-likelihood.

Torque - Torque is computed as the rotation of the tooth around the e-axis. The e-axis is orthogonal to the l and d-axes in FIG. 4.

Arch form parallelism - Two separate metrics may be computed - a per-tooth "local" arch form parallelism metric, and a per-arch "global" arch form parallelism metric. The steps to compute these two metrics are shown below:

    1. For each tooth in an arch, compute the distance to the opposing arch as follows:

        a. Identify the tooth origin, t
        b. Identify the origins of the two nearest neighbor teeth on the opposing arch, o1 and o2.
        c. Draw a line, l, joining o1 and o2
        d. Compute the 2D Euclidean distance in the x-y plane between t and l

    2. Compute the global arch form parallelism as the standard deviation of the distances computed in Step 1 for all teeth in an arch
    3. Compute the local arch form parallelism by computing the mean value of the distances computed in Step 1, then computing the difference between each tooth's distance and the mean value.

Arch form expansion - The arch form expansion metric is defined as the observed "d" axis translation with the malocclusion (initial) state as the reference state. The metric captures translation in both directions i.e. expansion and constriction with respect to the malocclusion. There are two different metrics based on this definition, a global and an individual tooth wise metric. To construct this metric, an arch form as a connected list of tooth origins is produced. The sum of piece wise linear inter-origin distances is measures as the arch form length. By measuring the arch form length of a given state and the malocclusion state, an overall expansion ration (global arch form expansion metric) can be calculated. This can be calculated separately for the mandible and maxilla.

To compute the individual metric per tooth, the displacement in the d direction is calculated between malocclusion and a given state. This is measured as just the difference between the d coordinate of the tooth in the current state and the d coordinate of the tooth in the malocclusion state.

Golden proportions metric:

Golden proportions are used to define how wide different teeth should appear when looking from the front. Teeth can be altered using IPR or positioned suitable to accomplish a good smile according to golden proportions. Tooth widths from a front view can be measured by projecting the teeth meshes on a plane perpendicular to the front view or global Y axis. Tooth widths can be measured as the distance between tooth edge points or corners in this projected space. Tooth widths can be directly used as metrics or tooth widths scaled by tooth width of central upper incisors can be used.

Bolton ratio:

Bolton ratio is an extension of golden proportions to all 12 front teeth 1st molar to 1st molar. It governs an overall length. The first molar to first molar length of mandible should be 91.3% of the maxillary 1st molar to 1st molar length.

This metric can be implemented using the sum of tooth widths in 3D space without taking any projections. The metric can be the ratio of the mandibular length to the maxillary length or vice versa. This can be explicitly penalized for being different from 0.913 or can be measured from approved setups as discussed in the next section.

Curve of Spee:

Curve of spee is a measure of curvature of the mandibular occlusal plane. This can be measured by first computing the curve joining the tooth origins of the mandible and then computing the curvature at each tooth origin in the curve. This measure is also indirectly represented by the individual tooth leveling metrics described earlier. The set of curvature values can be penalized for deviation from acceptable values of approved final setups as described in the next section.

**B: Conversion of metrics to scores**

Metrics may be converted to scores that represent their agreement or deviation from ideal values in a patient population. These scores can then be used to rate final setups and inform clinicians and/or automated systems about which metrics are currently in agreement with good final setups, suggesting that they do not need to be improved further, and which metrics are not in agreement with good final setups, suggesting that they need to be further refined.

First, baseline values for each of the metrics are learned from an ideal population of final setups. The ideal final setups may be further refined or even catered to specific practices or doctors (e.g., a separate set of ideal final setups for each doctor). All features are computed for each setup in the set of ideal final setups. For each metric, the median, $k^{th}$ percentile and $(100-k)^{th}$ percentile are computed. For concreteness, the k=20 case is described.

Once baseline values have been computed, metrics for new final setups can be converted to scores using the approach shown in the pseudo-code below:

$$\text{if metric\_value} > \text{ideal\_median:}$$
$$\text{score} = (\text{metric\_value} - \text{ideal\_median}) / (\text{ideal\_80}^{th}\text{\_percentile} - \text{ideal\_median})$$
$$\text{else:}$$
$$\text{score} = (\text{metric\_value} - \text{ideal\_median}) / (\text{ideal\_median} - \text{ideal\_20}^{th}\text{\_percentile})$$

A score between -1 and 1 indicates that the metric value is within the $20^{th}$ - $80^{th}$ percentile values of the metric in the ideal population, while a score greater than 1 or less than -1 indicates that the metric is outside of the normal range in the ideal population.

**C: Machine learning method to rate setups:**

The metrics and/or scores described above may be used to train a machine learning classifier to rate setups. First, baseline values for each metric and/or score are learned from a population of maloccluded and final setups. This is followed by an optional normalization step - this will make the features have 0 mean and unit variance. One class and/or multi-class classifiers are subsequently trained using cross validation to identify final setups. One class-classifiers could be, for example, one class Support Vector Machine (SVM), elliptical covariance estimator, or a Principal Components Analysis (PCA) reconstruction error based classifier. Multi-class classifiers could be, for example, decision trees, random forest, Adaboost classifier, Naive Bayes, or neural networks. Other classifiers are also possible.

**D: Use in automated final setup generation**

Scores and/or machine learning classifiers can be used for several tasks during automated final setup generation:

1. Selection of one or more initialization "goal" setups from a set of candidate initialization setups

Initialization "goal" setups represent setups that fix some issues with the initial malocclusion but still need to be optimized to result in an adequate final setup. These setups may allow for speed-up of the final setup search process by allowing for difficult series of movements to be accomplished upfront. Goal setups may be generated by performing simple operations (e.g., aligning and uprighting all teeth in the arch). Alternatively, they can be learned based on data from existing final setups.

Once a set of goal setups has been created, the best setup(s) may be selected by choosing the setup(s) that minimize one or more scores, or by choosing the setup(s) that a machine learning model rates as being most representative of a final setup.

## 2. Loss function for optimization

Final setups may be automatically created by iteratively adjusting the position of teeth to minimize a loss function. The loss function can be defined as a single metric or score, a linear or non-linear combination of metrics/scores, or the continuous output of a machine learning classifier. Output from a machine learning classifier may include, for example, the distance to a hyperplane in a SVM, the distance metric in a GMM, or a probability that the state is a member of the malocclusion or setup class in a two-class classifier.

## 3. Metric selection for optimization

The scores described in Section B indicate the deviation of metrics from an ideal final setup. A score between -1 and 1 indicates that the metric lies within ideal data, while values outside this range suggest that the metric lies outside of ideal values and should be further improved. Thus, scores can be used to select metrics in a setup that need to be further optimized. Conversely, scores can also be used to identify metrics that are currently in the acceptable range and that should not be increased during optimization.

## 4. Stopping criteria for optimization

Metrics, scores, or the output of a machine learning classifier can be used to evaluate the acceptability of a setup. If the setup lies within an acceptable range, iterative optimization can be terminated.

## 5. Selection of final setup from a set of candidate final setups

Final setup generation can be designed to produce multiple candidate final setups, from which a subset can be selected. The subset may include the single best scoring final setup or multiple final setups that achieve the best scores or scores above a threshold. Alternatively, scores can be used to identify a subset of final setups that represent certain tradeoffs in setup design. For example, if it is not feasible to design a final setup that achieves both good midline alignment and class relationship, two final setups could be identified that demonstrate these tradeoffs (i.e., one setup having a good class relationship score but bad midline alignment, while the other setup has a good midline alignment score but bad class relationship).

## E: Use in an interface

Metrics, scores, and/or machine learning classifiers can be used in interactive tools to assist clinicians during final setup design and evaluation:

### 1. Interactive tool for final setup design

Metrics could be computed and displayed to a technician or clinician during interactive setup design. The technician or clinician could use their clinical expertise to determine which metrics required further improvement and could perform the appropriate tooth movements to achieve these improvements. Such a tool could also be used to train new technicians in how to design an acceptable final setup.

Alternatively, scores for individual metrics and/or machine learning classifier output could be displayed. This would provide information about the severity of issues with setup design. The machine learning classifier would alert the clinician or technician if the setup did not closely resemble an ideal final setup. Scores would indicate which metrics needed to be further refined to achieve a "setup-like" arrangement of teeth.

### 2. Interface for final setup evaluation

Metrics, scores, and/or machine learning classifier output could be provided to a clinician and patient in a display interface alongside the final setup. This easily interpretable information would help the patient understand the final setup design and promote treatment acceptance.

### 3. Interface for setup selection from candidate final setups

Metrics, scores, and/or machine learning classifier output could be used to demonstrate trade-offs between multiple candidate final setups. This information could be displayed alongside several options for final setups along with additional factors (e.g., treatment time, treatment cost) to enable the patient to make an informed decision about treatment selection.

The following aspects are preferred embodiments of the present invention.

1. A computer-implemented method for generating one or more final setups for orthodontic treatment, comprising steps of:

receiving a digital 3D model of teeth in an initial state;
computing a scoring function based upon metrics related to the initial state to generate a corresponding score;
perturbing a state of the teeth through movement of a tooth or set of teeth within the digital 3D model of teeth to generate a perturbed state of the teeth; and
updating the state of the teeth based upon the score and the perturbed state of the teeth to generate one or more final setups representing a final state of the teeth after the orthodontic treatment.

2. The method of aspect 1, wherein the receiving step comprises receiving positions for each tooth in the digital 3D model of teeth.

3. The method of aspect 1, wherein the receiving step comprises receiving information about tooth landmarks for the teeth.

4. The method of aspect 1, wherein the receiving step comprises receiving information about extractions for the teeth.

5. The method of aspect 1, wherein the receiving step comprises receiving information about holding teeth during the orthodontic treatment.

6. The method of aspect 1, wherein the receiving step comprises receiving information about interproximal reduction for the teeth.

7. The method of aspect 1, wherein the receiving step comprises receiving information about limits on tooth movements.

8. The method of aspect 1, wherein the receiving step comprises receiving information about areas of focus.

9. The method of aspect 1, wherein the receiving step comprises receiving information about orthodontic qualities to maintain or improve.

10. The method of aspect 1, wherein the receiving step comprises receiving information about desired treatment duration.

11. The method of aspect 1, wherein the receiving step comprises receiving information about desired tooth or arch form changes.

12. The method of aspect 1, further comprising outputting positions and orientations and interproximal reduction amounts for the teeth for the final setup.

13. The method of aspect 1, wherein the computing step comprises computing as the scoring function

$$Score(X) = \sum_{i=1}^{n\_metrics} w_i P_i(x_i),$$

where X represents a vector of metrics computed for a state, $P_i$ is a penalty function that computes an error or penalty given a value of a metric and acceptable levels for that metric, and $w_i$ is a weight associated with the penalty for the metric.

14. The method of aspect 1, wherein the computing step comprises computing as the scoring function f(P(x)), where f is a function selected from one or more of linear, non-linear, and a probabilistic framework functions.

15. The method of aspect 1, wherein the perturbing step comprises translating a tooth or set of teeth within the digital 3D model of teeth to generate the perturbed state of the teeth.

16. The method of aspect 1, wherein the perturbing step comprises rotating a tooth or set of teeth within the digital 3D model of teeth to generate the perturbed state of the teeth.

17. The method of aspect 1, wherein the perturbing step comprises applying interproximal reduction to a tooth or set of teeth within the digital 3D model of teeth to generate the perturbed state of the teeth.

18. The method of aspect 1, wherein the perturbing step comprises a combination of one or more of the following: translating, rotating, and applying interproximal reduction to a tooth or set of teeth within the digital 3D model of teeth to generate the perturbed state of the teeth.

19. The method of aspect 1, wherein the perturbing step comprises computing a numerical gradient based upon different states of the teeth and using the computed gradient to minimize or maximize the scoring function.

20. The method of aspect 1, further comprising repeating the computing, perturbing, and updating for a maximum number of iterations.

21. The method of aspect 1, further comprising repeating the computing, perturbing, and updating for a number of iterations based upon termination criteria.

22. The method of aspect 1, wherein the receiving step comprises performing one or more of the following initialization methods to generate the initial state: unrotate; leveling; symmetric translations/rotations; parallel arches; ideal rotation; ideal arch form shape; expansion; anti-mesialization and distalization; edge/groove alignment; class relationship; crossbite/overjet; packing or gap closing; and direct mapping and machine learning.

23. The method of aspect 1, further comprising generating an initial final state of the teeth representing a state of the teeth after orthodontic treatment.

24. The method of aspect 1, wherein the initial state is a manually generated final setup or an automatically adjusted final setup.

25. A system for generating one or more final setups for orthodontic treatment, comprising a computing device for executing any of the methods of aspects 1-24.

26. A computer-implemented method for rating final setups for orthodontic treatment, comprising steps of:

receiving one or more final setups representing a proposed final state of teeth in a digital 3D model of teeth after orthodontic treatment; and
computing one or more scores representing a deviation of each of the one or more final setups from an ideal final setup.

27. The method of aspect 26, wherein the computing step comprises computing the metrics based upon a tooth coordinate system for the teeth.

28. The method of aspect 26, wherein the computing step comprises computing the metrics based upon landmarks on the teeth.

29. The method of aspect 26, wherein the computing step comprises computing the metrics based upon a tooth coordinate system for the teeth and landmarks on the teeth.

30. The method of aspect 26, further comprising displaying the one or more final setups and the corresponding score for each of the one or more final setups.

31. The method of aspect 26, further comprising selecting one or more final setups from a set of candidate final setups using the one or more scores.

32. The method of aspect 26, further comprising computing metrics for the one or more final setups and converting the metrics to the one or more scores.

33. The method of aspect 26, wherein the computing step comprises computing the one or more scores using a population statistics-based method.

34. The method of aspect 26, wherein the computing step comprises computing the one or more scores using a machine learning method.

35. A computer-implemented method for rating setups for orthodontic treatment, comprising steps of:

receiving one or more setups representing a proposed state of teeth in a digital 3D model of teeth after a particular stage of orthodontic treatment; and
computing one or more scores representing a deviation of each of the one or more setups from an ideal setup for the particular stage of orthodontic treatment.

36. A system for rating setups for orthodontic treatment, comprising a computing device for executing any of the methods of aspects 26-35.

**Claims**

1. A computer-implemented method of generating a final setup for orthodontic treatment using supervised machine learning for direct mapping, comprising:

receiving, by one or more computer processors, a training set of patient case data which includes malocclusion states and ground truth images representing final setup states for the teeth of one or more patients, wherein the final setup states represent orthodontic post-treatment positions of a person's teeth and the malocclusion states are pre-treatment positions of the teeth and are represented by digital 3D models of teeth in an initial state;
generating, by the one or more computer processors using a deep neural network, a generated output image;
computing, by the one or more computer processors, a distance between a generated output image and the ground truth image representing the final state using a loss function; and
minimizing, by the one or more computer processors, the distance to train, at least in part, the deep neural network.

2. The computer-implemented method of claim 1, wherein the deep neural network is trained to model the mapping between one or more malocclusions and respective final states.

3. The computer-implemented method of claim 1, wherein the deep neural network is a convolutional auto-encoder (CAE).

4. The computer-implemented method of claim 1, wherein the loss function is defined as one or more of: a single metric or score, a linear combination of metrics or scores, or a non-linear combination of metrics or scores, or the continuous output of a machine learning classifier.

5. The computer-implemented method of claim 1, wherein the digital 3D models of teeth are based on one or more intra-oral 3D scans.

6. The computer-implemented method of claim 4, wherein the metric comprises at least one of: alignment, edge alignment, buccolingual inclination, class relationship, leveling, mesial tilt, distal tilt, Torque, relative midline, absolute midline, one or more occlusal contacts, overbite, canine overbite, overjet, spacing, collision, contact, crossbite, bite symmetry, arch form shape, arch form symmetry, inter-arch contact, arch form parallelism, arch form expansion, golden proportions metric, Bolton ratio, or curve of spee.

7. The computer-implemented method of claim 4, wherein the score indicates the deviation of one or more metrics from an ideal final setup.

8. The computer-implemented method of claim 1, further comprising:

generating, by the one or more computer processors, two or more candidate final setups; and

selecting, by the one or more computer processors, a final setup from the generated candidate setups.

9. The computer-implemented method of claim 4, further comprising displaying, by the one or more computer processors and on an electronic display device, the one or more metrics, the one or more scores, or the one or more machine learning classifier outputs alongside the final setup.

10. The computer-implemented method of claim 1, wherein the training set of patient case data includes one or more of: tooth positions for every tooth in a maloccluded state, information about tooth landmarks, information about tooth coordinate systems, information about extractions, information about teeth whose positions are fixed during treatment, information about desired interproximal reduction, information about limits on tooth movements, information about areas of focus, information about orthodontic qualities that should be maintained or improved, information about desired treatment duration, or information about desired tooth or arch form changes.

11. The computer -implemented method of claim 10, wherein patient case data contains position and orientation information of the malocclusion states for every tooth in the arch form coordinate system.

12. The computer-implemented method of claim 1, wherein the generated output image includes at least one of: a position of one or more teeth, an orientation of one or more teeth, or an interproximal reduction quantity for one or more teeth.

13. The computer-implemented method of claim 1, wherein receiving, by one or more computer processors, a training set of patient case data which includes malocclusion states and ground truth images representing final setup states includes receiving the patient case data directly in a state space, transformation space, metric space or combination thereof.

*FIG. 1*

*FIG. 2*

*FIG. 3*

Archform Coordinate System:
Variable Definitions

p - point along S with archlength $\ell$

t - tooth origin, obtained by
translating up along d axis
by a distance d and then
translating along e axis
by a distance e (not shown)

*FIG. 4*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7956862 B
- US 7605817 B

- US 62712380